# EUROPEAN PATENT SPECIFICATION

(11) **EP 3 573 553 B1**
(45) Date of publication and mention of the grant of the patent: **04.11.2026**
(21) Application number: 18704661.0
(22) Date of filing: 23.01.2018
(51) Int. Cl.: A61B 17/86

(54) **POROUS FIXATION DEVICES AND METHOD OF MANUFACTURING**
PORÖSE FIXIERVORRICHTUNGEN UND HERSTELLUNGSVERFAHREN
DISPOSITIFS DE FIXATION POREUSE ET MÉTHODE DE FABRICATION

(30) Priority: 27.01.2017 US 201762451405 P
(43) Date of publication of application: 04.12.2019
(73) Proprietor: Zimmer, Inc., Warsaw, Indiana 46580 (US)
(72) Inventor: ROBY, Keith A., Jersey City, New Jersey 07307 (US); CHERNOSKY, John, Brick, New Jersey 08724 (US); LIEPINS, Imants, Asbury, New Jersey 08802 (US); COALE, Bradford J., Chester, New Jersey 07930 (US); MACKE, Jacob, Warsaw, Indiana 46580 (US)
(74) Representative: Allen, Caroline Margaret
(86) International application number: PCT/US2018/014776
(87) International publication number: WO 2018/140375

(56) References cited:
- US-A1- 2010 042 215
- US-A1- 2011 264 151
- US-A1- 2012 277 804
- US-A1- 2016 157 908

## Description

### PRIORITY APPLICATIONS

This application claims the benefit of priority to U.S. Provisional Patent Application Serial No. 62/451,405, filed January 27, 2017.

### FIELD OF THE DISCLOSURE

The present disclosure relates to surgical implant systems, including implants, instruments, and methods. Specifically, the present disclosure relates to porous screws and pegs, and methods of manufacturing the porous screws and pegs.

### BACKGROUND

Bone screws are used in various orthopedic surgical contexts to provide stability and fixation to the biological tissue. For example, bone screws are used in trauma applications, in joint replacements, or in bone plating applications. A primary concern with bone screws is to ensure that the screw adequately fixes to bone and does not back out of the bone during the life cycle of that device. Including porosity in certain areas of the screw can augment the fixation of the screw, particularly given variation in bone quality and properties. Existing screws suffer in one (1) or more of these areas, for example the screws might be susceptible to backing out and/or have poor bone fixation. US 2012/277804 discloses a threaded bone screw, the middle portions of its threading being coated with bone growth promoting agent while the peaks of the threading are devoid of said coating. US 2011/264151 relates to threaded bone screws having a shaft and threads, the shaft and portions of the threads being coated with hydroxyapatite or other material that promotes bone ingrowth and improves the bone-to-metal interface, the outer edge of the threads being devoid of said coating.

The present disclosure provides bone screws and pegs that improve on existing bone screws and pegs.

### SUMMARY

The invention provides a bone screw comprising a shaft having a thread, the thread having a thread core formed of a first biocompatible material, wherein the thread defines a root and a crest, a proximal thread face is disposed between the root and the crest on a first side of the thread, and a distal thread face is disposed between the root and the crest on a second side of the thread and a porous layer of a second biocompatible material which is a titanium alloy capable of being formed over the thread core of the shaft by additive manufacturing and covering only part of the proximal or distal thread face of the thread, wherein a portion of the thread core defines a crest of the thread, and the porous layer of the second biocompatible material is thinner in the area of the crest as compared to other areas of the porous layer of the second biocompatible material..

To better illustrate the bone disclosed herein, a non-limiting list of examples is provided here. Optionally, the first biocompatible material of the bone screw of can be a non-porous metal or metal alloy, and the thread core can be formed entirely of the first biocompatible material.

Optionally, the interstitial spaces can be formed between adjacent crests of the thread and at least some of the interstitial spaces can include a porous layer of the second biocompatible material.

Also described is a bone screw comprising a core formed of a first biocompatible material, the core having a distal threaded section, an intermediate section, and a proximal head section, and a porous sleeve covering the intermediate section of the core, the porous sleeve being composed of a second biocompatible material that has a porous architecture that replicates natural cancellous bone.

Optionally the first biocompatible material of the bone screw described above can be a non-porous metal or metal alloy, or a metal or metal alloy with a porosity that is lower than a porosity of the porous sleeve.

Optionally, the proximal head section of the bone screw described above can define a first diameter, the intermediate section can define a second diameter, and the distal threaded section can define a third diameter representing an outer diameter of the threads in the distal threaded section, and the second diameter can be smaller than the first and third diameters.

In a further aspect, the invention provides a method of manufacturing a bone screw as defined in claim 10.

Optionally, the second biocompatible material of the bone screw can have a porous architecture that replicates natural cancellous bone.

Optionally, the core of the bone screw can define interstitial spaces between adjacent crests of the thread, and the method further comprises covering part of a first of the interstitial spaces with a porous layer of the second biocompatible material using additive manufacturing.

### BRIEF DESCRIPTION OF THE FIGURES

The above-mentioned and other features and advantages of this disclosure, and the manner of attaining them, will become more apparent and the disclosure itself will be better understood by reference to the following description of examples taken in conjunction with the accompanying drawings, wherein:
Figs. 1A-B are side and top perspective views of a porous screw according to an example of the disclosure.
Figs. 2A-B are side and top perspective views of a first porous peg according to an example of the disclosure.
Figs. 3A-B are side and top perspective views of a second porous peg according to another example of the disclosure.
Figs. 4A-B are side and top perspective views of a third porous peg according to yet another example of the disclosure.
Fig. 5A is a side view of a porous screw according to an example of the disclosure.
Fig. 5B is a cross-sectional view of the porous screw of Fig. 5A.
Figs. 5C-F are close-up detailed views of a distal section (Fig. 5C, 5F), threads (Fig. 5D), and a head (Fig. 5E) of the porous screw of Fig. 5A.
Figs. 6A-B are cross-sectional views of an alternative example of the porous screw of Fig. 5A.
Fig. 7A illustrates OsseoTi^{®}, a material that can be used to form any of the screws or pegs disclosed herein, as discussed in more detail below, and Fig. 7B illustrates human cancellous bone.
Fig. 8 illustrates the architecture of Trabecular Metal^{™}, a material produced by Zimmer, Inc.

Corresponding reference characters indicate corresponding parts throughout the several views. The exemplifications set out herein illustrate examples of the disclosure, and such exemplifications are not to be construed as limiting the scope of the disclosure any manner.

### DETAILED DESCRIPTION

In describing the examples of the disclosure illustrated and to be described with respect to the drawings, specific terminology will be used for the sake of clarity. However, the examples are not intended to be limited to any specific terms used herein, and it is to be understood that each specific term includes all technical equivalents.

Various porous fixation devices, such as porous screws and pegs, are disclosed herein. The porous screws and pegs, in some cases, can be formed through additive manufacturing techniques, such that porosity can be embedded on and/or intermeshed with threading on the screw or peg. In an example, porosity can be formed on the threads of the screw or peg themselves and/or on other portions of the screw or peg. The screws or pegs can be used in various contexts, in an example for fixation of a portion of a joint prosthesis to bone, fixation of plating to bone, or in trauma applications.

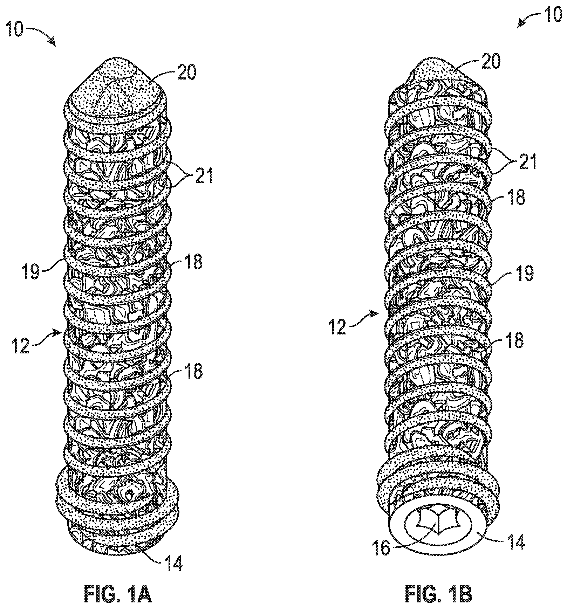

Referring to Figs. 1A-B, an example of a porous screw 10 is disclosed. Porous screw 10 can include a shaft 12 that is threaded along part or all of its length and a head 14. Head 14 can include threads, in an example for interacting with a threaded hole in a bone plate, joint replacement, or other prosthesis. Alternatively, head 14 can be non-threaded or smooth or have threads for engaging bone instead of a prosthesis. Head 14 can further include a drive engagement 16, for instance a hexagonal-shaped recess configured to engage with a hexagonal-shaped male portion of a driving instrument (not shown). Drive engagement 16 can take any shape (e.g., triangular, oval, diamond, square, hexagonal, etc.) and/or have internal threading or press-fit features for engaging with the male part of the driving instrument. Screw 10 can also have a distal tip 20 forming a point for insertion into bone, which in an example can include self-tapping features (not shown), such as a cutting flute(s). The cutting flute(s) would allow screw 10 to self-drill into bone and might not require a pre-tapping operation to be performed for insertion of screw 10 into bone.

The entirety of screw 10 can be formed through an additive manufacturing process, such as 3D printing, selective laser sintering, selective laser melting, or any other additive manufacturing process. As such, screw 10 can be manufactured with different materials at different regions and/or have porosity in certain regions, but not in others. For instance, shaft 12 can have a core that is formed of a first material by way of additive manufacturing, and outer surfaces of shaft 12 can be formed of a second material through the additive manufacturing process. The first material can be titanium or another suitable biocompatible metal or metal alloy (optionally non-porous), and the second material can be a porous material, such as OsseoTi^{®}, which is commercially available from Biomet Manufacturing, LLC (Warsaw, Ind., USA). Thus, porous sections 18 can be formed over the core of shaft 12 through additive manufacturing. In an example, porous sections 18 can be formed of OsseoTi^{®}. This can create a situation in which the core of screw 10 is a solid biocompatible metal or metal alloy, for example titanium, and outer surfaces of screw 10 include porous sections 18 that stimulate bone ingrowth when screw 10 is implanted in bone. In this and other examples, the core of screw 10 can be non-porous or it can have a porosity that is lower than the porosity of the outer surfaces of screw 10 formed of OsseoTi^{®}. For instance, the porosity of the core can be approximately be anywere between about zero to fifty percent (0-50%) porous. In addition, the core of shaft 12 can be formed with threading on the core, and then outer surfaces of shaft 12 can include OsseoTi^{®} formed over part or all of the threading.

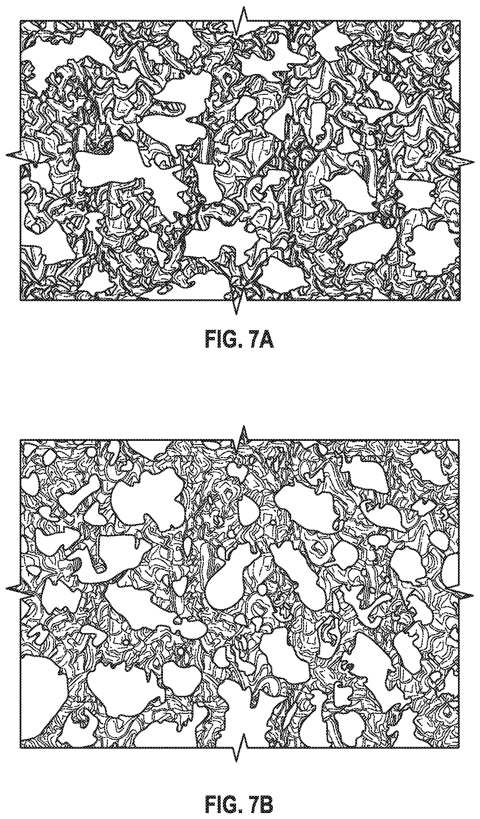

Briefly, OsseoTi^{®} can be a titanium alloy, in an example Ti6Al4V, that is highly biocompatible, has high corrosion resistance, and includes a highly interconnected porous architecture that mimics the porous structure of human cancellous bone. Figs. 7A-B illustrate the architecture of OsseoTi^{®} as compared to human cancellous bone, and it is apparent that the architecture of OsseoTi^{®} mimics that of cancellous bone. OsseoTi^{®} can therefore enhance bone integration and ingrowth and can be used on select areas of screw 10 to improve osseointegration. In one exemplary implementation, the OsseoTi^{®} porous metal construct can include a porosity of 70%.

Because screw 10 can be formed using an additive manufacturing process, the porosity present on screw 10 can be formed directly on the threads of shaft 12 themselves and/or on interstitial spaces 21 between the threads. In an example, using 3D printing or another additive manufacturing technique, the second material (e.g., OsseoTi^{®}) can be deposited layer-by-layer on the core of screw 10, thereby attributing porosity to the thread surfaces themselves and/or interstitial spaces 21 between the threads. The core can also be formed through the same additive manufacturing technique, but using the first material detailed above (*e.g.*, a non-porous metal or metal alloy, or a metal or metal alloy with a porosity that is lower than that of OsseoTi^{®}). In an example, the porosity of the second material (e.g., OsseoTi^{®}) can be anywhere between fifty to ninety-five percent (50-95%) porous.

**In** any of the prior examples of screw 10 or in any of the screws and/or pegs discussed below, when threads are described as themselves being formed of OsseoTi^{®}, it can mean that the entire thread structure from root to crest (e.g., the entire thread depth) can be formed of OsseoTi^{®}, or that a layer of OsseoTi^{®} can be formed along part, substantially all, or an entirety of the surfaces defining the thread. In the latter case, the threads covered with a layer of OsseoTi^{®} can be threading present on the core of shaft 12, which can be formed of a biocompatible metal or metal alloy (optionally non-porous), as discussed above.

More particularly, the threads of the above and below examples can be said to define a root, a crest, a proximal thread face between the root and crest on a first side of the thread, and a distal thread face between the root and the crest on a second side of the thread. Crest 19 is shown in Figs. 1A-B, but the root and proximal/distal thread faces are hidden from view. In certain instances, a layer of OsseoTi^{®} can be formed on part of the proximal and/or distal thread faces defining the thread, and a portion of the thread that is not formed of OsseoTi^{®} can extend from the OsseoTi^{®} layer. The protruding part can be crest 19 of the threads, which presents a sharp thread edge that can be formed from a non-porous biocompatible material (e.g., a non-porous metal or metal alloy) or a material that has a porosity that is lower than that of OsseoTi^{®}. This results from the fact that the core of screw 10 can be formed of the first biocompatible material discussed above, with threading, and the core can then be covered via additive manufacturing with OsseoTi^{®}. Since in an example only part of the proximal and/or distal thread faces are covered with OsseoTi^{®}, a portion of the core of screw 10 that defines crest 19 of the thread can protrude from or beyond the OsseoTi^{®} layer. This creates a situation in which a non-porous, sharp crest 19 can be presented at the outermost parts of the threads for digging into bone. In addition, porous OsseoTi^{®} can be formed on non-crest areas of the threads and/or at interstitial spaces 21 to facilitate bone ingrowth into screw 10 at the same time.

OsseoTi^{®} can cover roughly ten percent to about ninety percent (10-90%) of the proximal and/or distal thread faces of each thread, or it can cover anywhere between about thirty percent to about sixty percent (30-60%) of the proximal and/or distal thread faces. Thus, as noted above, crest 19 of the threads can be somewhat exposed and define a sharp edge formed of the material of the core of shaft 12, which in an example is the first biocompatible material discussed above. Crests 19 can therefore each present a sharp, non-porous edge that is more suited to dig into bone than an ill-defined porous edge.

**In** the above examples, it can be said that each thread can have a thread core formed of a non-OsseoTi^{®} material (e.g., a non-porous metal or metal alloy) and a layer of OsseoTi^{®} can cover part or all of the thread core to establish a porous layer covering part or all of the thread. In certain instances, interstitial spaces 21 can also include a porous material (e.g., OsseoTi^{®}) that covers part or all of each interstitial space 21. For instance, the core of shaft 12 and the threads thereof can be formed through additive manufacturing out of a first biocompatible material (e.g., a non-porous metal or metal alloy), and porous OsseoTi^{®} can be deposited in interstitial spaces 21 to cover part or all of spaces 21. The OsseoTi^{®} can have a thickness that is anywhere between about ten percent to about ninety percent (10-90%) of the depth of adjacent threads bordering the particular interstitial space 21, or it can have a thickness that is anywhere between about thirty percent to about sixty percent (30-60%) of the depth of the adjacent threads. If only part of an interstitial space 21 is covered, the remaining areas can be exposed parts of the core of shaft 12, which can be formed of the first biocompatible material.

In yet another example, the thread can itself include a thread core formed of the first material discussed above *(e.g.,* a non-porous metal or metal alloy) and alternating layers of a porous material (e.g., OsseoTi^{®}) can be formed on the proximal and/or distal thread faces of the thread, such that the thread core is exposed between layers of the porous material. In addition, interstitial spaces 21 between the threads in any of the above examples can include a porous material (e.g., OsseoTi^{®}) or another material *(e.g.,* a non-porous metal or metal alloy). Thus, many combinations are possible and are envisioned by the present disclosure and are applicable to any of the examples discussed above and/or below with reference to the figures or otherwise.

**In** even further examples, it is contemplated that porosity can vary or be uniform along the length of shaft 12 of screw 10. The porosity could for instance be greater at a distal section of shaft 12 as compared to the porosity at an intermediate and/or a proximal section of shaft 12, or vice versa. Porous OsseoTi^{®} can also be present along only part of the threads and/or interstitial spaces 21 of shaft 12 while not being present on other areas of shaft 12. For instance, OsseoTi^{®} might be present only on the threads and/or interstitial spaces 21 of a distal section of shaft 12, an intermediate section of shaft 12, a proximal section of shaft 12, or any combination of the foregoing sections. The other sections not utilizing OsseoTi^{®} might have traditional non-porous threads in these examples. Indeed, the non-porous threads can be formed by the core of screw 10 *(e.g.,* a non-porous metal or metal alloy).

The depicted embodiment in Figs. 1A-B includes threading along substantially the entire length of shaft 12, but in alternate examples only part of shaft 12 is threaded while other parts are non-threaded or smooth. For instance, a proximal section of shaft 12 can include threading that is non-porous (*e.g.*, a non-porous metal or metal alloy) or porous (e.g., OsseoTi^{®}), an intermediate section of shaft 12 can be non-threaded and/or smooth, and a distal section of screw 10 can include threading that is non-porous *(e.g.,* a non-porous metal or metal alloy) or porous (e.g., OsseoTi^{®}), in any combination. As contemplated above, interstitial spaces 21 between the threads in the proximal and/or distal sections can also include porosity (e.g., be formed of OsseoTi^{®}). In a further example (discussed in more detail below with reference to pegs 110, 210, 310), the intermediate section of screw 10 can be formed of a porous material (e.g., OsseoTi^{®}) and have a design similar to shafts 112, 212, 312. It is also contemplated that screw 10 can be cannulated through its entire length so that a K-wire can be received in shaft 12.

Further, the threads in the proximal and distal sections of shaft 12 can have different pitches. For example, the threads in the proximal section can have a finer pitch than threads in the distal section of screw 10. This can enable screw 10 to be used as a compression screw in, for instance, fracture applications.

In another example, screw 10 can have threads along the length of its shaft 12, but the pitch of the threads can be variable instead of constant (the depicted example of Figs. 1A-B have constant-pitch threads). For instance, shaft 12 can have a continuous thread with a pitch that changes from a coarser pitch near a distal section of shaft 12 to a finer pitch near a proximal section of shaft 12. Such a variable pitch screw 10 can also be used in compression-type applications to compress bone parts together. In an alternate example, the variable pitch continuous thread can have a finer pitch near the distal section of shaft 12 changing to a coarser pitch near the proximal section of shaft 12. Further, the variable pitch continuous thread can also extend along only part of shaft 12 instead of the entire length of shaft 12, as discussed above.

Screw 10 can further include a root diameter for shaft 12 that is tapered from tip 20 to adjacent head 14, or it can include a constant-diameter root. Figs. 1A-B show screw 10 with a constant-diameter root for shaft 12, except at tip 20 of course. In addition, in these and other examples, an outer diameter of the threads on shaft 12 can be constant along part or all of shaft 12, or alternatively variable (e.g., tapered). In an example, an outer diameter of the threads can be tapered in a proximal section, intermediate section, and/or distal section of shaft 12. Further, the screw threads on shaft 12 can be cortical threads, cancellous threads, or some combination of both to suit the application at hand. Cortical threads are characterized by a pitch that is finer than that of cancellous threads, which are coarser. Cortical threads with their finer pitch are more suited to dig into cortical bone, while cancellous threads with their coarser pitch are more suited to dig into cancellous bone. In an example, screw 10 can include cancellous threads at a distal section of shaft 12 and cortical threads at a proximal section of shaft 12 and/or on head 14 to engage with cortical bone.

After forming screw 10 through additive manufacturing, screw 10 can undergo post-processing. As an example, screw 10 can be subject to heat treating, machining, electrical discharge machining (EDM) processes, or other post-processing techniques. Such post-processing can improve thread sharpness for the threads on shaft 12, form self-tapping features on the threads, and/or improve drive engagement 16. At non-porous parts of screw 10, screw 10 can also be polished, bead blasted, or undergo other post-processing as well. It has been found, however, that such techniques can be, but not necessarily are, unsuitable for post-processing porous sections 18 of screw 10.

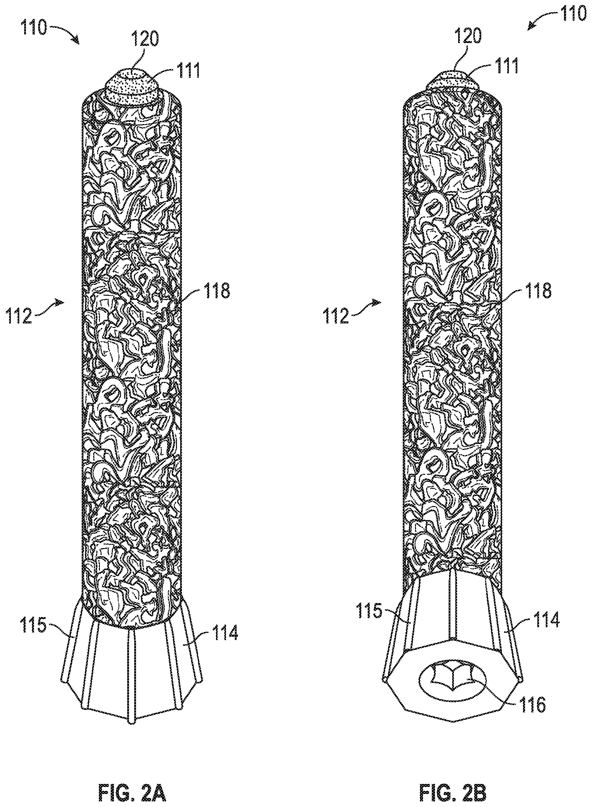

Figs. 2A-4B illustrate various embodiments of porous pegs 110, 210, 310. Each of porous pegs 110, 210, 310 can include a porous shaft 112, 212, 312 and a non-porous core 111, 211, 311. As with screw 10, pegs 110, 210, 310 can be formed using any of the aforementioned additive manufacturing techniques, and cores 111, 211, 311 and shafts 112, 212, 31 `? can be formed from any of the first and second materials discussed above. As an example, cores 111, 211, 311 can be formed of a non-porous metal or metal alloy (e.g., titanium), and shafts 112, 212, 312 can be formed using additive manufacturing from OsseoTi^{®}. Thus, shafts 112, 212, 312 can facilitate positive bone in growth via the use of OsseoTi^{®} when pegs 110, 210, 310 are implanted in bone. Each of pegs 110, 2 10, 310 can further include a head 114, 214, 314 that has a drive engagement 116, 216, 316. Drive engagements 116, 216, 316 can take the form of any of the aforementioned disclosed drive engagements 16 for screw 10.

Referring to Figs. 2A-B, peg 110 can include a shaft 112 that is non-threaded along its entire length and is partially or substantially entirely formed of OsseoTi^{®}. In an example, shaft 112 can have a substantially constant diameter along a majority of its length, or substantially all of its length, and a tapered tip 120. Core 111 can, in some cases, protrude slightly from the OsseoTi^{®} of shaft 112 at tip 120, as shown. In an example, head 114 of peg 110 can also be tapered and can include one (1) or more protruding ribs 115. Ribs 115 can extend axially along part or substantially all of head 114 so as to substantially coincide with a longitudinal axis of shaft 112.

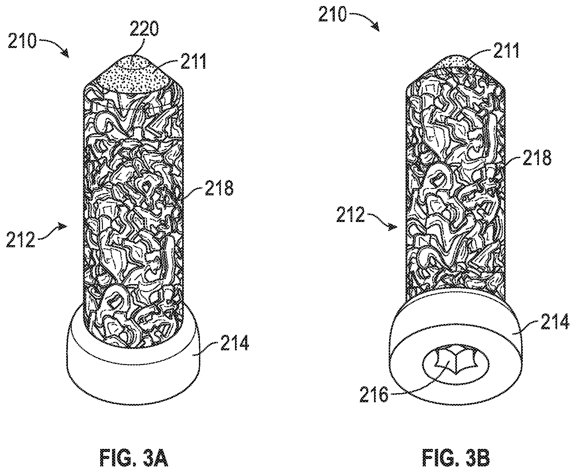

Peg 210, as shown in Figs. 3A-B, is similar to peg 110 but can instead include a head 214 that is smooth and/or forms a rounded shape. Like peg 110, peg 210 can have a shaft 212 with a substantially constant diameter along a majority or substantially all of its length and a tapered tip 220. In addition, core 211 can also protrude slightly from the OssecTi^{®} of shaft 212 at tip 220, as shown.

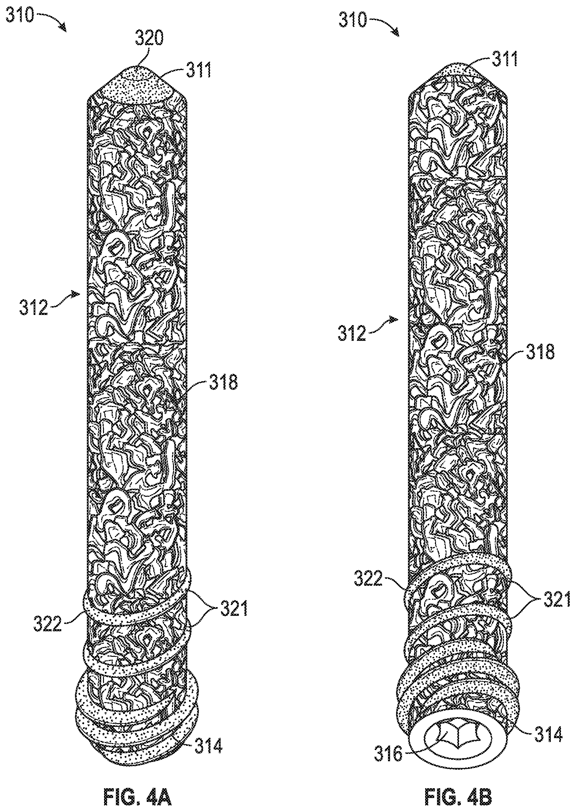

Peg 310 is illustrated in Figs. 4A-B. Peg 310 can include a head 314 that is threaded for engagement with a threaded hole *(e.g.,* in a bone plate, joint prosthesis, or other prosthetic component) or bone. In addition, shaft 312 of peg 310 can include one (1) or more threads 322, in an example, for engaging bone when peg 310 is inserted into bone. As shown, and similar to screw 10, because peg 310 is formed by way of an additive manufacturing process, threads 322 themselves can include porosity as threads 322 themselves can be formed from OsseoTi^{®}, which replicates the architecture of natural cancellous bone. In addition, like screw 10, interstitial spaces 321 on shaft 312 between threads 322 can be formed from OsseoTi^{®} and therefore include the porosity and attributes of that material, as described in detail above. As with screw 10, peg 310 can also be subject to post processing in the manner described above (e.g., particularly as to threads 322, which can be machined, sharpened, and/or include self-tapping features).

Alternative examples of screw 10 (not shown) can also be provided that incorporate features of pegs 110, 210, and/or 310. For instance, screw 10 can have threads along part of shaft 12, non-threaded OsseoTi^{®} sections along other parts of shaft 12 *(e.g.,* as with shafts 112, 212, 312 of pegs 110, 210, 310), and/or further threads formed on shaft 12 that provide boundaries for the non-threaded OsseoTi^{®} sections. In an example, an intermediate section of shaft 12 can include a non-threaded OsseoTi^{®} section similar to pegs 110, 210, 310, while a proximal and/or distal section of shaft 12 can include threads, similar to those shown in Figs. 1A-B. Alternatively, a non-threaded OsseoTi^{®} section can be positioned at a proximal section of screw 10 immediately adjacent head 14, and a remainder of shaft 12 can be threaded similar to as shown in Figs. 1A-B. Even further, a proximal and intermediate section of shaft 12 can be threaded similar to as shown in Figs. 1A-B, while a distal section of shaft 12 can include a non-threaded OsseoTi^{®} section similar to shafts 112, 212, 312. In another example, non-threaded OsseoTi^{®} sections and threaded sections can alternate along shaft 12, such that a non-threaded OsseoTi^{®} section is between two (2) threaded sections and a threaded section is between two (2) non-threaded OsseoTi^{®} sections. As such, any combination of threaded sections and non-threaded OsseoTi^{®} sections that are similar to those of pegs 110, 210, 310 can be utilized along shaft 12 to create an alternate screw 10 with improved fixation characteristics. It should be noted that although threaded sections are not described above as OsseoTi^{®} sections, such threaded sections can be formed of OsseoTi^{®} as described in connection with screw 10. Alternatively, in another example, the threaded sections can be formed of a non-porous biocompatible metal or metal alloy (e.g., titanium), and/or certain threaded sections can be formed of OsseoTi^{®} while other threaded sections are formed of a non-porous biocompatible metal or metal alloy.

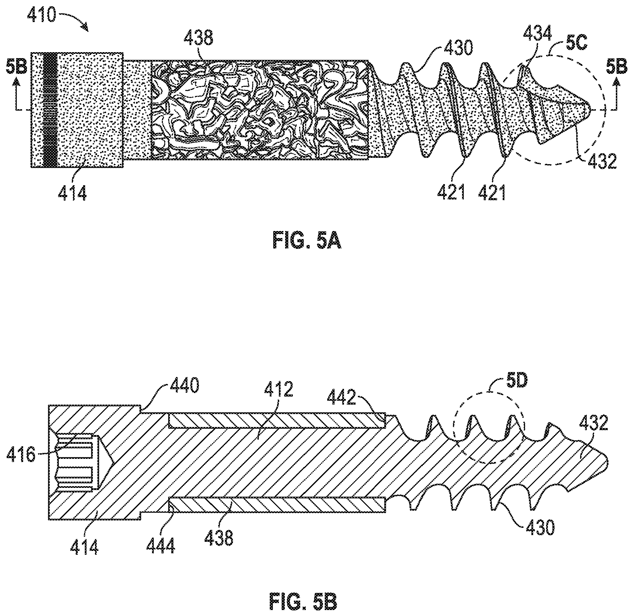

Another example of a porous screw 410 is shown in Figs. 5A-F. Similar to porous screw 10, porous screw 410 can be formed by way of any of the additive manufacturing techniques disclosed above. Thus, it is to be understood that the different components of porous screw 410 discussed below can all be formed through additive manufacturing.

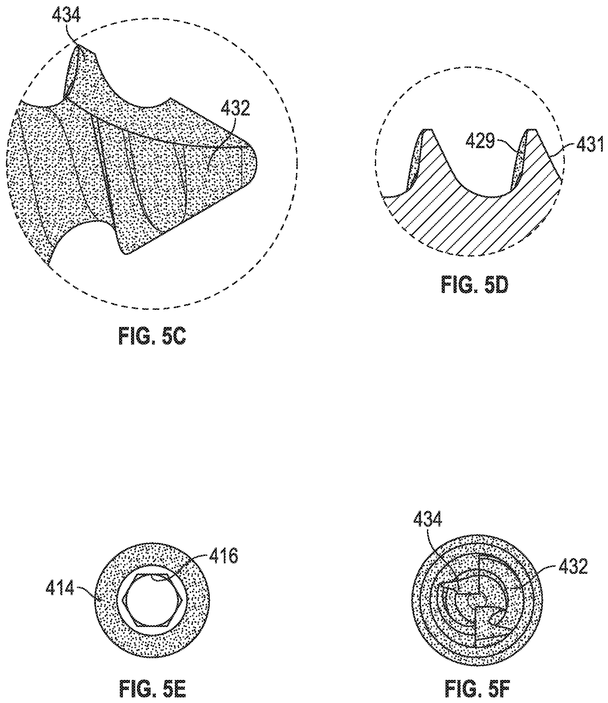

Porous screw 410 can include a shaft 412 and a head 414 with a drive engagement 416. In an example, head 414 can be integral or monolithic with shaft 412 by virtue of forming shaft 412 and head 414 through an additive manufacturing process. Shaft 412 can have a distal threaded section 430 leading to a tip 432 that forms a point. As shown in Figs. 5C and 5F, tip 432 can have one (1) or more cutting flutes 434 configured to cut into bone as screw 410 is screwed into bone, for example two (2) cutting flutes 434. In an example, flutes 434 can form self-tapping features on screw 410. Cutting flutes 434 can be disposed on opposite sides of shaft 412 of screw 410. As shown in Fig. 5D, the threads of threaded section 430 can also be defined by a lower angled thread face 431 and an upper substantially-perpendicular or alternatively substantially-transverse thread face 429. In an example, an angle between faces 429, 439 can be approximately thirty two degrees (32°) plus/minus two and a half degrees (+/- 2.5°). Further, in another example, the pitch of the threads in threaded section 430 can be constant or variable. For instance, the pitch could vary from a greater pitch near tip 432 to a smaller pitch as one approaches a proximal part of threaded section 430. This could have the effect of creating compression between bone parts as screw 410 is screwed into bone.

A sleeve 438 can be formed over a portion of shaft 412, as shown in Figs. 5A-B. In an example, sleeve 438 can be formed using OsseoTi^{®} and have the porous structure of OsseoTi^{®} material, as described above. By contrast, in an example shaft 412 and head 414 can be formed of a material that is different from OsseoTi^{®}. For instance, shaft 412 and head 414 can be formed of a non-porous metal or metal alloy as detailed above, or alternatively shaft 412 and head 414 can be formed of a porous metal or metal alloy having a different architecture and/or porosity from that of OsseoTi^{®}. In yet another example, the threads on distal threaded section 430 can be formed of OsseoTi^{®} in the manner set forth above for screw 10, such that the OsseoTi^{®} porous architecture is embedded in and/or forms the threads of distal threaded section 430. In this example, an inner core of distal threaded section 430 can be formed of a non-porous material *(e.g.,* a non-porous metal or metal alloy), while the threads and/or interstitial spaces 421 between the threads can be formed using OsseoTi^{®} so as to have an architecture that replicates cancellous bone. Thus, distal threaded section 430 and/or interstitial spaces 421 can utilize the OsseoTi^{®} features of the numerous examples discussed above with reference to screw 10.

Sleeve 438 can be retained between a portion of a thread on distal threaded section 430 and head 414, as shown in Fig. 5B. In particular, a proximal-most thread of distal threaded section 430 can form a step 442, and a step 444 can be formed adjacent head 414. A distance between steps 442, 444 can be approximately the same as a length of sleeve 438 and can contact end portions of sleeve 438 so as to retain sleeve 428 longitudinally. In another example, the distance between steps 442, 444 can be slightly larger or smaller than the length of sleeve 438. If the distance is larger than sleeve 438, sleeve 438 can be formed on shaft 412 so that a slight amount of movement therebetween is permitted (e.g., when screw 410 is loaded). In another example, sleeve 438 is fixed relative to shaft 412 so that substantially no movement occurs therebetween when screw 410 is loaded. As stated previously, sleeve 438 can be formed of OsseoTi^{®} that is deposited on shaft 412 through an additive manufacturing process. By virtue of the fact that additive manufacturing is used, shaft 412 and head 414 can also form an integral or monolithic structure and still an intermediate portion of shaft 412 can fit within sleeve 438.

Head 414 of screw 410 also can include a step 440, such that head 414 is enlarged and/or has a diameter that is larger than a diameter of shaft 412. As explained in the context of a surgical method below, step 440 can be used to engage with a portion of a hole in a prosthetic device (e.g., another step in the hole) so that screw 410 can be used to fix the prosthetic device to bone. In an example, the hole in the prosthetic device can be countersunk in the prosthetic device *(e.g.,* bone plate, joint component, etc.), such that head 414 can fit in the countersink and step 440 can contact a step in the hole and enable screw 410 to fix the prosthetic device to bone. Drive engagement 416 of screw 410 can also be any of the drive engagements discussed previously, as an example a hexagonal-shaped recess (shown in Fig. 5E), triangular, oval, square, or any other shape allowing non-rotational engagement with a male part of a driving instrument.

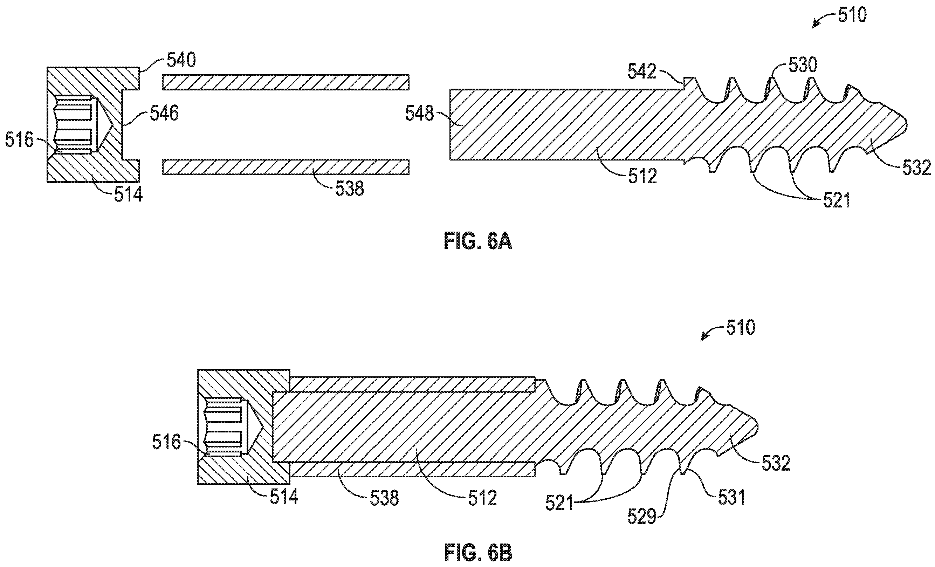

An alternate example of screw 410 - screw 510 - is shown in Figs. 6A-B. As with prior examples of the disclosure, like reference numerals refer to like elements in this example and, predominantly, only the differences between screws 410, 510 will be discussed. In this example, some or all of the components of screw 510 may be formed by traditional manufacturing techniques and not additive manufacturing, although as discussed below some alternate examples can provide for the use of additive manufacturing of certain components.

As shown in Fig. 6B, screw 510 can include a separate head 514 and shaft 512 structure. Head 514 can have a recess 546 that can be cylindrical, hexagonal, square, or another shape, which can mate with a projecting part 548 of shaft 512. A fixed connection can be established between recess 546 and projecting part 548 as, for example, by laser welding, press fitting, a threaded (*e.g.*, screw and nut) engagement, or any other fixed engagement. In addition, the engagement can be non-rotational, such that head 514 is non-rotationally fixed relative to shaft 512. In an example, head 514 and/or shaft 512 can be produced through traditional manufacturing techniques and not through additive manufacturing. Further, head 514 and/or shaft 512 can also be formed of a non-porous material *(e.g.,* a non-porous metal or metal alloy), or a material that has a porosity that is less than that of OsseoTi^{®}. In an alternate example, however, head 514 and/or shaft 512 can be produced through additive manufacturing using the materials and/or having any of the material attributes discussed above with reference to screw 410. As an example, shaft 512 can be formed via additive manufacturing and/or from different materials with different porosities, or can be produced via additive manufacturing and be a non-porous single material *(e.g.,* a biocompatible metal or metal alloy). If porosity is desired, for example, shaft 512 can utilize additive manufacturing to form a non-porous core of a first material *(e.g.,* a metal or metal alloy) and a porous distal threaded section 530. In this example, OsseoTi^{®} can be used to form outer layers of distal threaded section 530, and/or the threads themselves can be formed of OsseoTi^{®}. Similar to screws 10, 410, interstitial spaces 511 can also be formed of OsseoTi^{®}. Thus, distal threaded section 530 can be predominantly formed of OsseoTi^{®} to create porosity in that area and replicate the architecture of cancellous bone.

Screw 510 can also include a separate sleeve 538 that, in an example, is formed through traditional, non-additive manufacturing techniques. In an example, sleeve 538 is formed of a highly porous biomaterial useful as a bone substitute and/or cell and tissue receptive material. An example of such a material is produced using Trabecular Metal^{™} technology generally available from Zimmer, Inc., of Warsaw, Ind. Trabecular Metal^{™} is a trademark of Zimmer Technology, Inc. Such a material may be formed from a reticulated vitreous carbon foam substrate which is infiltrated and coated with a biocompatible metal, such as tantalum, etc., by a chemical vapor deposition ("CVD") process in the manner disclosed in detail in U.S. Pat. No. 5,282,861. Other metals such as niobium, or alloys of tantalum and niobium with one another or with other metals may also be used.

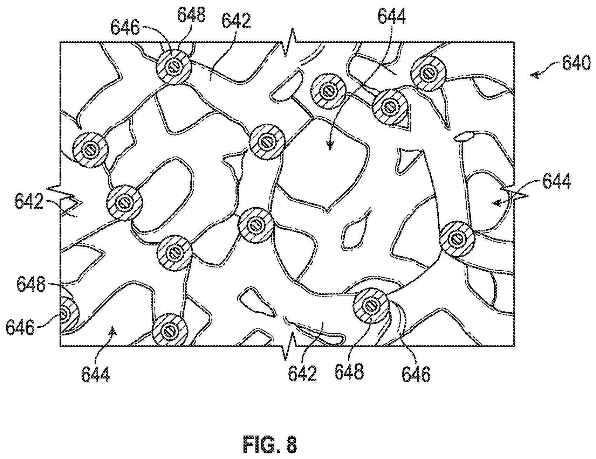

Generally, as shown in Fig. 8, porous tantalum structure 640 includes a large plurality of ligaments 642 defining open spaces 644 therebetween, with each ligament 642 generally including a carbon core 646 covered by a thin film of metal 648 such as tantalum, for example. The open spaces 644 between ligaments 642 form a matrix of continuous channels having no dead ends, such that growth of cancellous bone through porous tantalum structure 640 is uninhibited. The porous tantalum may include up to seventy-five to eighty-five percent (75-85%) or more void space therein. Thus, porous tantalum is a lightweight, strong porous structure which is substantially uniform and consistent in composition, and closely resembles the structure of natural cancellous bone, thereby providing a matrix into which cancellous bone may grow to anchor screw 510 into the surrounding bone of a patient.

Porous tantalum structure 640 may be made in a variety of densities in order to selectively tailor the structure for particular applications. In particular, as discussed in the above-mentioned U.S. Pat. No. 5,282,861, the porous tantalum may be fabricated to virtually any desired porosity and pore size, and can thus be matched with the surrounding natural bone in order to provide an improved matrix for bone ingrowth and mineralization. This includes a gradation of pore size on a single implant such that pores are larger on a first end section to match cancellous bone and smaller on a second end section to match cortical bone, or even to receive soft tissue ingrowth. Such gradation of pore size can be applied to sleeve 538. Also, the porous tantalum could be made denser with fewer pores in areas of high mechanical stress. Instead of smaller pores in the tantalum, this can also be accomplished by filling all or some of the pores with a solid material which is described in further detail below.

The following is applicable to all examples set forth in the present disclosure where porosity is used on any of the disclosed screws or pegs. To provide additional initial mechanical strength and stability to the porous structure (e.g., OsseoTi^{®} or Trabecular Metal^{™} portions of the disclosed screws or pegs), the porous structure may be infiltrated with filler material such as a non-resorbable polymer or a resorbable polymer. Examples of non-resorbable polymers for infiltration of the porous structure can include a polyaryl ether ketone (PAEK), such as polyether ketone ketone (PEKK), polyether ether ketone (PEEK), polyether ketone ether ketone ketone (PEKEKK), polymethylacrylate (PMMA), polyetherimide, polysulfone, and polyphenolsulfone.

Examples of resorbable polymers may include Polylactide (PLA), Polyglycolic acid (PGA), polylactic co-glycolic acid (PLGA), polyhydroxybutyrate (PI-FB), polyhydroxyvalerate (PHV), Poly-L-lactide (PLLA), and copolymers thereof, polycaprolactone, polyanhydrides, and polyorthoesters.

The pores of the porous parts disclosed herein may also be filled with other materials, such as bone growth proteins, antibiotics, or human derived, animal derived, or synthetic bone grafting materials. Each of the materials or combinations of any of these materials may provide a clinical advantage to the porous portion.

It should also be noted, for the screws and/or pegs of Figs. 1A-4B, that instead of OsseoTi^{®} being used as the applicable porous material, Trabecular Metal^{™} could be used. In these examples, the Trabecular Metal^{™} can be bonded, welded, and/or cold formed to the non-porous core of the screw and/or peg.

The screws and/or pegs disclosed herein can be used in various orthopedic surgical methods. As an example, the screws and pegs disclosed herein can be used to fix prosthetic components (e.g., a bone plate, acetabular cup, glenoid component, etc.) to bone. For instance, a hole(s) can extend through the prosthetic component and any screw or peg disclosed herein can be inserted through the hole to fix the prosthetic component to the bone. In these examples, the various porous parts of the above screws and pegs can be arranged to sit within cancellous and/or cortical bone and act to improve bone ingrowth into the relevant screw or peg and improve fixation. In certain examples, therefore, the disclosed screws and pegs are suitable for use in situations where poor bone quality is observed. The disclosed screws and pegs can be used in these instances as the porous parts thereof, with or without screw threads, can improve fixation of the relevant screw or peg to bone. In addition, if screw threads are utilized in the aforementioned porous parts of the screw or peg, additional fixation characteristics can be experienced while not sacrificing bone ingrowth (*e.g.*, since porosity is present on the thread itself and/or the interstitial spaces between threads, as discussed above).

Another application for certain of the screws disclosed herein, such as screw 10, is in trauma applications. For instance, certain versions of screw 10 can be used in trauma applications as compression screws to reduce fractures and bring bone parts defining the fracture(s) together. These versions of screw 10 typically include threads of differing pitches in different areas of shaft 12, which can facilitate compression of bone parts across a fⁱ⁻acture. As an example, some versions of screw 10 have coarser threads disposed in a distal section of shaft 12 and finer threads disposed in a proximal section of shaft 12. These versions of screw 10 can act to cause compression across a fracture and bring bone parts together. In particular, the coarser threads can advance faster than the finer threads and act to pull a distal bone fragment towards a proximal bone fragment. According to the above examples, these versions of screw 10 can have a continuous, variable pitch thread or alternatively can have a coarse thread in a distal section of shaft 12, an unthreaded or smooth intermediate section, and a finer thread in a proximal section of shaft 12. Further, the unthreaded or smooth intermediate section of screw 10 can be formed of porous OsseoTi^{®}, and can be arranged to straddle the fracture(s) line and/or reside in natural cancellous bone so that appropriate bone ingrowth occurs in the trauma repair. In addition, the porous threads of screw 10 (e.g., covered with OsseoTi^{®}) can also reside in natural cancellous bone and/or cortical bone to facilitate bone ingrowth. Thus, examples of screw 10 disclosed herein can be used in trauma applications, such as fracture repair. The porosity of screw 10, in particular its porous threads, can act to cause positive bone ingrowth into screw 10 and improved its usability as a fracture-repair device.

Another application for the screws and/or pegs disclosed herein is in spinal applications. For instance, any of the screws or pegs disclosed herein can be used with a cortical bone plate or as a pedicle screw for use in connecting spinal rods to adjacent vertebral bodies. In the case of a pedicle screw, any of the aforementioned screws or pegs can be designed with a head 14, 114, 214, 314, 414, 514 that can be received in a tulip part of the pedicle screw. Such a screw or peg can be designed so that shaft 12, 112, 212, 312, 412, 512 is polyaxially movable relative to the tulip portion of the pedicle screw, and is capable of being locked (*e.g.*, via a set screw) at a specific angle relative to the tulip portion. Prior to locking *(e.g.,* with a set screw), a suitable rod or other orthopedic implant capable of spanning adjacent vertebral bodies can be connected to one (1) or more of the pedicle screws. Thus, the screws and pegs can be used as pedicle screws and attached to a rod other orthopedic device capable of spanning two (2) or more vertebral bodies so as to be able to secure the bodies in position relative to each other, for example in the case of a fusion procedure. If a fusion procedure is being conducted, a suitable intervertebral implant and/or bone graft can be placed in the intervertebral space and the pedicle screws of the above examples used to fix the adjacent vertebral bodies to help affect stable fusion. Pedicle screws incorporating the features of the above-discussed screws and/or pegs can experience greater fixation in combination with positive bone ingrowth due to the porous features on the disclosed screws and/or pegs.

Certain of the screws disclosed herein, such as screw 10, can also be used in arthrodesis procedures to fuse joints. Screw 10 can be designed to have cortical-threaded sections where appropriate, and/or cancellous-threaded sections where appropriate, with or without porosity as discussed above on the thread itself and/or in the interstitial spaces between threads. Such a screw 10 can engage cancellous and/or cortical bone portions *(e.g.,* in the context of an arth^{r}odesis procedure). As mentioned above, cancellous threads are characterized by a coarser pitch and cortical threads are characterized by a finer pitch *(e.g.,* a pitch of anywhere between about).

It will be readily understood to those skilled in the art that various other changes in the details, material, and arrangements of the parts and method stages which have been described and illustrated in order to explain the nature of the inventive subject matter can be made without departing from the F';UV:IJw J scope of the inventive subject matter as expressed in the subjoined claims.

## Claims

1. A bone screw (10, 410. 510) comprising:
a shaft (12, 412, ₅12) having a thread, the thread having a thread core formed of a first biocompatible material, wherein the thread defines a root and a crest (19), a proximal thread face (429, ₅29) is disposed between the root and the crest on a first side of the thread, and a distal thread face (431, ₅₃1) is disposed between the root and the crest on a second side of the thread; and
a porous layer of a second biocompatible material which is a titanium alloy capable of being formed over the thread core of the shaft by additive manufacturing and covering only part of the proximal thread face or distal thread face of the thread
wherein a portion of the thread core defines a crest of the thread, and the porous layer of the second biocompatible material is thinner in the area of the crest as compared to other areas of the porous layer of the second biocompatible material.

2. The bone screw (10, 410. 510) of claim 1, wherein the first biocompatible material is a non-porous metal or metal alloy, and the thread core is formed entirely of the first biocompatible material.

3. The bone screw (10, 410. 510) of any of claims 1-2, wherein interstitial spaces (21, 421, ₅21) are formed between adjacent crests of the thread and at least some of the interstitial spaces include a porous layer of the second biocompatible material.

4. The bone screw (10, 410. 510) of any of claims 1-3, wherein the second biocompatible material has a porous architecture that replicates natural cancellous bone.

5. The bone screw of (to, 410. 510) any of claims 1-4, wherein the shaft (12, 412, ₅12) comprises a non-porous core formed of the first biocompatible material.

6. The bone screw of (10) any of claims 1-₅, further comprising a threaded head (¹4).

7. The bone screw (10, 410. 510) of any of claims 1-6, wherein the shaft (12, 412, ₅12) has a proximal section, an intermediate section, and a distal section, and wherein the proximal section is threaded with a thread having a first pitch, the intermediate section is unthreaded, and the distal section is threaded with a thread having a second pitch that is greater than the first pitch.

8. The bone screw (10, 410. 510) of claim ₇, wherein the intermediate section is at least partly covered by a porous layer of the second biocompatible material.

9. The bone screw (10, 410. 510) of any of claims 1-8, wherein a portion of the thread core defines a crest of the thread, and the crest is exposed and not covered with a porous layer of the second biocompatible material so as to be configured to contact and dig into bone.

10. A method of manufacturing the bone screw (10, 410. 510) according to claim 1 comprising:
forming the core; and
covering only part of the proximal or distal thread face, by way of additive manufacturing, with the porous layer of a second biocompatible material which is a titanium alloy.

11. The method of claim 10, further comprising covering anywhere between about 10-90% of the proximal or distal thread face, by way of additive manufacturing, with the porous layer of the second biocompatible material.

12. The method of any of claims 10 to 11, wherein a portion of the crest of the thread is exposed and not covered by the porous layer of the second biocompatible material.

13. The method of any of claims 10 to 12, wherein the second biocompatible material has a porous architecture that replicates natural cancellous bone.

14. The method of any of claims 10 to 13, wherein the core defines interstitial spaces (21, 421, ₅21) between adjacent crests of the thread, and the method further comprises covering part of a first of the interstitial spaces with a porous layer of the second biocompatible material using additive manufacturing.

## Patentansprüche

1. Knochenschraube (10, 410, 510), umfassend:
eine Welle (12, 412, 512), die ein Gewinde aufweist, wobei das Gewinde einen aus einem ersten biokompatiblen Material gebildeten Gewindekern aufweist, wobei das Gewinde einen Fuß und eine Spitze (19) definiert, eine proximale Gewindefläche (429, 529) auf einer ersten Seite des Gewindes zwischen dem Fuß und der Spitze angeordnet ist und eine distale Gewindefläche (431, 531) auf einer zweiten Seite des Gewindes zwischen dem Fuß und der Spitze angeordnet ist; und
eine poröse Schicht aus einem zweiten biokompatiblen Material, bei dem es sich um eine Titanlegierung handelt, die dazu in der Lage ist, durch additive Herstellung über dem Gewindekern der Welle gebildet zu werden und nur einen Teil der proximalen Gewindefläche oder distalen Gewindefläche des Gewindes zu bedecken,
wobei ein Abschnitt des Gewindekerns eine Spitze des Gewindes definiert und die poröse Schicht aus dem zweiten biokompatiblen Material in dem Bereich der Spitze im Vergleich zu anderen Bereichen der porösen Schicht des zweiten biokompatiblen Materials dünner ist.

2. Knochenschraube (10, 410, 510) nach Anspruch 1, wobei das erste biokompatible Material ein nicht poröses Metall oder eine nicht poröse Metalllegierung ist und der Gewindekern vollständig aus dem ersten biokompatiblen Material gebildet ist.

3. Knochenschraube (10, 410, 510) nach einem der Ansprüche 1-2, wobei Zwischenräume (21, 421, 521) zwischen benachbarten Spitzen des Gewindes gebildet sind und mindestens einige der Zwischenräume eine poröse Schicht des zweiten biokompatiblen Materials beinhalten.

4. Knochenschraube (10, 410, 510) nach einem der Ansprüche 1-3, wobei das zweite biokompatible Material eine poröse Architektur aufweist, die natürliche Spongiosa nachbildet.

5. Knochenschraube (10, 410, 510) nach einem der Ansprüche 1-4, wobei die Welle (12, 412, 512) einen nicht porösen Kern umfasst, der aus dem ersten biokompatiblen Material gebildet ist.

6. Knochenschraube (10) nach einem der Ansprüche 1-5, ferner umfassend einen Gewindekopf (14).

7. Knochenschraube (10, 410, 510) nach einem der Ansprüche 1-6, wobei die Welle (12, 412, 512) einen proximalen Teilabschnitt, einen Zwischenteilabschnitt und einen distalen Teilabschnitt aufweist und wobei der proximale Teilabschnitt mit einem Gewinde gewunden ist, das eine erste Steigung aufweist, der Zwischenteilabschnitt kein Gewinde aufweist und der distale Teilabschnitt mit einem Gewinde gewunden ist, das eine zweite Steigung aufweist, die größer als die erste Steigung ist.

8. Knochenschraube (10, 410, 510) nach Anspruch 7, wobei der Zwischenteilabschnitt mindestens teilweise von einer porösen Schicht aus dem zweiten biokompatiblen Material bedeckt ist.

9. Knochenschraube (10, 410, 510) nach einem der Ansprüche 1-8, wobei ein Abschnitt des Gewindekerns eine Spitze des Gewindes definiert und die Spitze freigelegt und nicht mit einer porösen Schicht aus dem zweiten biokompatiblen Material bedeckt ist, um dazu konfiguriert zu sein, mit dem Knochen in Kontakt zu kommen und sich in diesen einzugraben.

10. Verfahren zum Herstellen der Knochenschraube (10, 410, 510) nach Anspruch 1, umfassend:
Bilden des Kerns; und
Bedecken nur eines Teils der proximalen oder distalen Gewindefläche mittels additiver Herstellung mit der porösen Schicht aus einem zweiten biokompatiblen Material, das eine Titanlegierung ist.

11. Verfahren nach Anspruch 10, ferner umfassend Bedecken zwischen etwa 10-90 % der proximalen oder distalen Gewindefläche mittels additiver Herstellung mit der porösen Schicht aus dem zweiten biokompatiblen Material.

12. Verfahren nach einem der Ansprüche 10 bis 11, wobei ein Abschnitt der Spitze des Gewindes freigelegt und nicht von der porösen Schicht aus dem zweiten biokompatiblen Material bedeckt ist.

13. Verfahren nach einem der Ansprüche 10 bis 12, wobei das zweite biokompatible Material eine poröse Architektur aufweist, die natürliche Spongiosa nachbildet.

14. Verfahren nach einem der Ansprüche 10 bis 13, wobei der Kern Zwischenräume (21, 421, 521) zwischen benachbarten Spitzen des Gewindes definiert und das Verfahren ferner Bedecken eines Teils eines ersten der Zwischenräume mit einer porösen Schicht aus dem zweiten biokompatiblen Material unter Verwendung von additiver Herstellung umfasst.

## Revendications

1. Vis à os (10, 410, 510) comprenant :
une tige (12, 412, 512) possédant un filetage, le filetage présentant un noyau de filetage formé d'un premier matériau biocompatible, ledit filetage définissant une racine et une crête (19), une face de filetage proximale (429, 529) étant disposée entre la racine et la crête sur un premier côté du filetage, et une face de filetage distale (431, 531) étant disposée entre la racine et la crête sur un second côté du filetage ; et
une couche poreuse d'un second matériau biocompatible qui est un alliage de titane pouvant être formée sur le noyau du filetage de la tige par fabrication additive et ne recouvrant qu'une partie de la face proximale du filetage ou de la face distale du filetage,
une partie du noyau du filetage définissant une crête du filetage, et ladite couche poreuse du second matériau biocompatible étant plus mince dans la zone de la crête par rapport à d'autres zones de la couche poreuse du second matériau biocompatible.

2. Vis à os (10, 410, 510) selon la revendication 1, ledit premier matériau biocompatible étant un métal ou un alliage métallique non poreux, et ledit noyau du filetage étant entièrement formé du premier matériau biocompatible.

3. Vis à os (10, 410, 510) selon l'une quelconque des revendications 1 à 2, des espaces interstitiels (21, 421, 521) étant formés entre des crêtes adjacentes du filetage et au moins certains des espaces interstitiels comprenant une couche poreuse du second matériau biocompatible.

4. Vis à os (10, 410, 510) selon l'une quelconque des revendications 1 à 3, ledit second matériau biocompatible présentant une architecture poreuse qui reproduit un os spongieux naturel.

5. Vis à os (10, 410, 510) selon l'une quelconque des revendications 1 à 4, ladite tige (12, 412, 512) comprenant un noyau non poreux formé du premier matériau biocompatible.

6. Vis à os (10) selon l'une quelconque des revendications 1 à 5, comprenant en outre une tête filetée (14).

7. Vis à os (10, 410, 510) selon l'une quelconque des revendications 1 à 6, ladite tige (12, 412, 512) comportant une section proximale, une section intermédiaire et une section distale, et ladite section proximale étant filetée avec un filetage présentant un premier pas, ladite section intermédiaire étant non filetée, et ladite section distale étant filetée avec un filetage présentant un second pas qui est supérieur au premier pas.

8. Vis à os (10, 410, 510) selon la revendication 7, ladite section intermédiaire étant au moins partiellement recouverte par une couche poreuse du second matériau biocompatible.

9. Vis à os (10, 410, 510) selon l'une quelconque des revendications 1 à 8, une partie du noyau du filetage définissant une crête du filetage, et ladite crête étant exposée et non recouverte d'une couche poreuse du second matériau biocompatible de manière à être conçue pour entrer en contact et creuser dans l'os.

10. Procédé de fabrication de la vis à os (10, 410, 510) selon la revendication 1, comprenant :
la formation du noyau ; et
le recouvrement de seulement une partie de la face proximale ou distale du filetage, par fabrication additive, avec la couche poreuse d'un second matériau biocompatible qui est un alliage de titane.

11. Procédé selon la revendication 10, comprenant en outre le recouvrement d'environ 10 à 90 % de la face de filetage proximale ou distale, par fabrication additive, avec la couche poreuse du second matériau biocompatible.

12. Procédé selon l'une quelconque des revendications 10 à 11, une partie de la crête du filetage étant exposée et non recouverte par la couche poreuse du second matériau biocompatible.

13. Procédé selon l'une quelconque des revendications 10 à 12, ledit second matériau biocompatible présentant une architecture poreuse qui reproduit un os spongieux naturel.

14. Procédé selon l'une quelconque des revendications 10 à 13, ledit noyau définissant des espaces interstitiels (21, 421, 521) entre des crêtes adjacentes du filetage, et ledit procédé comprenant en outre le recouvrement d'une partie d'un premier des espaces interstitiels avec une couche poreuse du second matériau biocompatible en utilisant une fabrication additive.
